# EUROPEAN PATENT APPLICATION

(11) **EP 4 670 620 A1**
(43) Date of publication of application: **31.12.2025**
(21) Application number: 23933127.5
(22) Date of filing: 11.04.2023
(51) Int. Cl.: A61B 5/00, A61B 18/20, A61B 18/12, A61N 7/02

(54) **ENERGY-BASED APPARATUS, METHOD, SYSTEM, AND PROGRAM FOR GENERATING COAGULATION SPOTS AS ASYMMETRIC ARRANGEMENT PATTERN**

(71) Applicant: Ahn, Ga Ram, Seoul 06909 (KR)
(72) Inventor: Ahn, Ga Ram, Seoul 06909 (KR)
(74) Representative: dompatent
(86) International application number: PCT/KR2023/004845
(87) International publication number: WO 2024/214837

(57) **Abstract**

The present disclosure may include a communication part for performing communication with an image capturing device for capturing an image of the skin; and a processor for controlling an operation related to asymmetrical area contraction of the skin, wherein the processor includes: an analysis module for analyzing a treatment area within a skin image received from the image capturing device through the communication part; a determination module for determining, when multiple contraction areas are formed within the treatment area, locations of respective contraction centers with respect to neighboring contraction areas so that the neighboring contraction areas are formed adjacent each other without overlapping; an adjustment module for adjusting a focus location of an energy emission part to face a location of each of the contraction centers; and a control module for controlling the energy emission part so that each coagulation spot corresponding to the location of each of the contraction centers is generated in an asymmetric arrangement pattern through the energy emission part.

## Description

### [TECHNICAL FIELD]

The present disclosure relates to energy-based device, method, system, and program for generating coagulation spots in an asymmetric arrangement pattern.

### [BACKGROUND ART]

Nowadays, the number of consumers who visit the dermatology clinic is increasing due to the saggy skin.

The consumer with the saggy skin is undergoing the facial wrinkle lifting procedure through an energy-based device.

The energy-based device forms a coagulation spot by applying energy to a target point of the reticular dermis layer. In this case, the reticular dermis layer which is within a range where a coagulation phenomenon occurs around a target point contracts in a direction facing the corresponding coagulation spot in a plane parallel to the skin surface. The energy-based device may induce contraction of the skin area when a plurality of coagulation spots are formed within a narrow range.

However, because a conventional energy-based device is used by the operator's experience and intuition, it is impossible to contract the skin area as expected whenever the procedure is performed.

In addition, the conventional energy-based device fails to contract to the skin area and shape optimized depending on anatomical location information of a treatment zone.

Accordingly, research on an improved device capable of consistently contracting the skin area regardless of the operator's experience and intuition and contracting the treatment zone to the skin area optimized depending on the anatomical location information of the treatment zone is continuously conducted.

### [DETAILED DESCRIPTION OF THE INVENTION]

### [TECHINICAL PROBLEM]

An embodiment of the present disclosure may contract the skin area as expected regardless of the operator's experience and intuition.

In addition, an embodiment of the present disclosure may contract a treatment zone to the skin area and shape optimized depending on anatomical location information of the treatment zone.

The problems to be solved by the present disclosure are not limited to the problems mentioned above, and any other problems not mentioned will be clearly understood by one skilled in the art from the following description.

### [TECHNICAL SOLUTION]

According to an aspect of the present disclosure, an energy-based device which generate coagulation spots in an asymmetric arrangement pattern may include a communication part that performs communication with an image capturing device capturing an image of a skin, and a processor that controls an operation associated with asymmetrical area contraction of the skin. The processor may include an analysis module that analyzes a treatment zone in the skin image received from the image capturing device, through the communication part, a determination module that, when a plurality of contraction areas are formed in the treatment zone, determines locations of contraction centers of contraction areas neighboring to each other such that the neighboring contraction areas do not overlap each other and are formed adjacent to each other, an adjustment module that adjusts a focus location of an energy emission part, so as to face the location of each of the contraction centers, and a control module that controls the energy emission part such that coagulation spots respectively corresponding to the locations of the contraction centers are generated in the asymmetric arrangement pattern through the energy emission part.

In addition, the analysis module may further analyze an image of a grid formed in the treatment zone.

Also, when the plurality of contraction areas are formed in the grid, the determination module may further determine locations of contraction centers of the neighboring contraction areas, such that the neighboring contraction areas do not overlap each other and are formed adjacent to each other.

Furthermore, when the plurality of contraction areas are formed, the determination module may determine locations of contraction centers of the some contraction areas vertically neighboring to each other, such that some contraction areas vertically neighboring to each other do not overlap and are formed adjacent to each other.

Besides, when the plurality of contraction areas are formed, the determination module may determine locations of contraction centers of the some contraction areas horizontally neighboring to each other, such that some contraction areas horizontally neighboring to each other do not overlap and are formed adjacent to each other.

Moreover, when the plurality of contraction areas are formed, the determination module may determine locations of contraction centers of the some contraction areas vertically and horizontally neighboring to each other such that some contraction areas vertically and horizontally neighboring to each other do not overlap and are formed adjacent to each other.

In addition, a shape of the grid includes at least ones of a plurality of points, a plurality of lines, and a plurality of surfaces.

According to another aspect of the present disclosure, a method of generating coagulation spots in an asymmetric arrangement pattern, which is performed by an energy-based device, may include receiving an image of a skin from an image capturing device, through a communication part of the energy-based device, analyzing a treatment zone in the skin image, through an analysis module of the energy-based device, determining, when a plurality of contraction areas are formed in the treatment zone, locations of contraction centers of contraction areas neighboring to each other such that the neighboring contraction areas do not overlap each other and are formed adjacent to each other, through a determination module of the energy-based device, adjusting a focus location of an energy emission part of the energy-based device to face the location of each of the contraction centers, through an adjustment module of the energy-based device, and controlling the energy emission part such that coagulation spots respectively corresponding to the locations of the contraction centers are generated in the asymmetric arrangement pattern, through a control module of the energy-based device.

In addition, the analyzing further analyzes an image of a grid formed in the treatment zone, through the analysis module.

Also, when the plurality of contraction areas are formed in the grid, the determining may further determine locations of contraction centers of the neighboring contraction areas, such that the neighboring contraction areas do not overlap each other and are formed adjacent to each other, through the determination module.

Furthermore, when the plurality of contraction areas are formed, the determining may determine locations of contraction centers of the some contraction areas vertically neighboring to each other, such that some contraction areas vertically neighboring to each other do not overlap and are formed adjacent to each other, through the determination module.

Besides, when the plurality of contraction areas are formed, the determining may determine locations of contraction centers of the some contraction areas horizontally neighboring to each other, such that some contraction areas horizontally neighboring to each other do not overlap and are formed adjacent to each other, through the determination module.

Moreover, when the plurality of contraction areas are formed, the determining may determine locations of contraction centers of the some contraction areas vertically and horizontally neighboring to each other, such that some contraction areas vertically and horizontally neighboring to each other do not overlap and are formed adjacent to each other, through the determination module.

According to another aspect of the present disclosure, a system which generates coagulation spots in an asymmetric arrangement pattern may include an image capturing device that captures an image of a skin, and an energy-based device that performs communication with the image capturing device and controls an operation associated with asymmetrical area contraction of the skin. The energy-based device may include an analysis module that analyzes a treatment zone in the skin image received from the image capturing device, a determination module that, when a plurality of contraction areas are formed in the treatment zone, determines locations of contraction centers of contraction areas neighboring to each other such that the neighboring contraction areas do not overlap each other and are formed adjacent to each other, an adjustment module that adjusts a focus location of an energy emission part, so as to face the location of each of the contraction centers, and a control module that controls the energy emission part such that coagulation spots respectively corresponding to the locations of the contraction centers are generated in the asymmetric arrangement pattern, through the energy emission part.

In addition, the determination module may further determine a contraction area for optimized asymmetric contraction by using at least one of contraction area information for each age group, contraction area information for each face shape, and contraction area information for each skin condition, which are based on an artificial intelligence model.

Besides, a computer-readable recording medium may be further provided which is coupled to a computer, which is hardware, to store a program for executing the method of generating the coagulation spots in the asymmetric arrangement pattern.

In addition, a computer-readable recording medium may be further provided which stores a computer program for executing a method for implementing the present disclosure.

### [ ADVANTAGEOUS EFFECTS OF THE INVENTION]

According to the above problem solving means of the present disclosure, there is provided an effect capable of contracting the skin area as expected, regardless of the operator's experience and intuition.

In addition, according to the above problem solving means of the present disclosure, there is provided an effect capable of contracting a treatment zone to the skin area and shape optimized depending on anatomical location information of the treatment zone.

The effects of the present disclosure are not limited to the effects mentioned above, and any other effects not mentioned will be clearly understood by one skilled in the art from the following description.

### [ DESCRIPTION OF THE DRAWINGS]

FIG. 1 is a diagram illustrating an example of a system generating coagulation spots in an asymmetric arrangement pattern, according to the present disclosure.
FIG. 2 illustrates a configuration of an energy-based device of FIG. 1.
FIG. 3 is a flowchart illustrating a method of generating coagulation spots in an asymmetric arrangement pattern, according to the present disclosure.
FIG. 4 is a diagram illustrating an example of a process of analyzing a treatment zone in a skin image through an analysis module of FIG. 2.
FIGS. 5 and 6 are diagrams illustrating an example of a process of analyzing an image of a grid formed in a treatment zone through an analysis module.
FIG. 7 is a diagram illustrating an example of a process of determining asymmetrical contraction by adjusting an asymmetrical pattern depending on anatomical location information of a treatment zone through a determination module of FIG. 2.
FIGS. 8 to 17 are diagrams illustrating an example of a process of setting location values of contraction centers by using a grid in a square treatment zone from among an asymmetric pattern of FIG. 7.
FIG. 18 is a diagram illustrating a process of setting location values of contraction centers to have a contraction area having asymmetric arrangement without a grid in a square treatment zone of an asymmetric pattern of FIG. 7.
FIG. 19 is a diagram illustrating an example of a process of emitting energy such that coagulation spots are generated in an asymmetric arrangement pattern through an energy-based device of FIG. 1.

### [ BEST MODE]

The same or similar reference numerals/signs refer to the same or similar components throughout the present disclosure. The present disclosure does not describe all components of embodiments, and the general content in the technical field to which the present invention pertains or the duplicated content between the embodiments is omitted. A component which is termed as "part", "module", "member", or "block" used in the specification may be implemented by using software or hardware. Depending on embodiments, a plurality of "parts", "modules", "members", and "blocks" may be implemented with a single component, or a single "part", "module", "member", and "block" may include a plurality of components.

Throughout the specification, when a part is described as being "connected to" another part, it includes the case where they are indirectly connected, as well as the case where they are directly connected, and the indirect connection includes the connection through a wireless communication network.

Also, when it is mentioned that a part "includes" any component, this means that any other component(s) may be further included, rather than excluding any other component(s), unless otherwise stated.

Throughout the specification, when a first member is described as being "on" a second member, it includes the case where a third member is present between the first and second members, as well as the case where the first member is in contact with the second member.

The terms such as "first", "second", etc. are used to distinguish one component from another component, and the components are not limited by the above terms.

A singular expression includes a plural expression, unless there are obvious exceptions in the context.

In each step, a reference sign is used for convenience of description, and the reference sign does not describe the order of respective steps. Each step may be carried out to be different from the specified order unless the specific order is clearly stated in the context.

Below, the operation principle and embodiments of the present disclosure will be described with reference to the accompanying drawings.

The external change which we perceive as aging is not symmetrical expansion, but it is mainly vertically downward asymmetric relaxation due to a gradual decrease in skin elasticity and the influence of continuous gravity.

A plurality of contraction centers formed during the procedure by the energy-based device currently in use are distributed with an even density.

For example, the distribution of the plurality of contraction centers, which is in the shape of a square grid, is formed of intersections of a plurality of parallel lines orthogonal at the same interval or is formed of a plurality of points, each of which has the same distances until points adjacent in the left, right, up, and down directions.

The arrangement of the even density has the effect of symmetrically contracting the treatment zone. For example, the arrangement of the even density, which is in the shape of a square grid, may have the effect of contracting a square treatment zone to a smaller square.

Accordingly, because the energy-based device generating energy in an arrangement pattern of the even density is used by the operator's experience and intuition, the energy-based device fails to contract the skin area as expected whenever the procedure is performed. Also, the energy-based device which generates energy in the arrangement pattern of the even density fails to provide the contraction to the skin area and shape optimized depending on anatomical location information of the treatment zone.

According to the present disclosure, as the arrangement of a plurality of contraction centers is generated in an asymmetric arrangement pattern other than an arrangement pattern of an even density, the skin area is contracted as expected regardless of the operator's experience and intuition, and the contraction to the skin area and shape optimized depending on anatomical location information of the treatment zone is made.

To this end, as a technical solution, a system according to the present disclosure, which generates coagulation spots in an asymmetric arrangement pattern, may analyze a treatment zone in a skin image received from an image capturing device, may determine, when a plurality of contraction areas are formed in the treatment zone, locations of contraction centers of neighboring contraction areas such that the neighboring contraction areas do not overlap each other and are formed adjacent to each other, may adjust a focus location of an energy emission part to face a location of each of the contraction centers, and may control the energy emission part such that coagulation spots respectively corresponding to the locations of the contraction centers are generated in an asymmetric arrangement pattern, through the energy emission part.

Below, a system according to the present disclosure, which generates coagulation spots in an asymmetric arrangement pattern, will be described in detail.

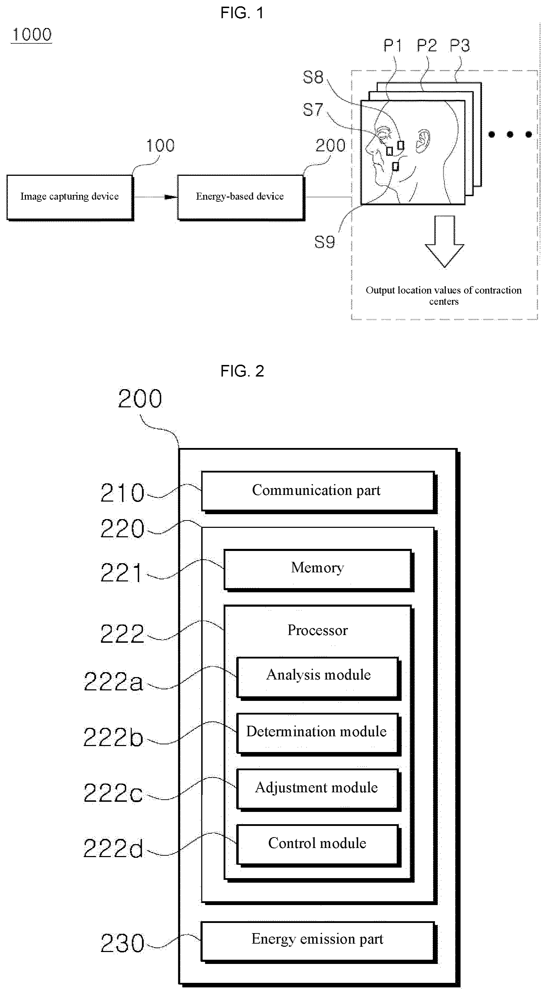

FIG. 1 is a diagram illustrating an example of a system generating coagulation spots in an asymmetric arrangement pattern, according to the present disclosure. FIG. 2 illustrates a configuration of an energy-based device of FIG. 1.

Referring to FIGS. 1 and 2, a system 1000 may include an image capturing device 100 and an energy-based device 200.

The image capturing device 100 may obtain an image of the skin and may transmit the image to the energy-based device 200. The image capturing device 100 may be a CCD camera and may further include a polarized filter for reducing the diffuse reflection. Alternatively, the image capturing device 100 may be an optical microscope into which lenses are inserted to increase resolution or may be a dermatoscope which is in contact with the skin to obtain an enlarged image.

The energy-based device 200 may perform an operation associated with asymmetrical area contraction of the skin. The energy-based device 200 may locally heat the dermis to cause contraction of the skin and may be a laser, a microneedle radio frequency, a non-invasive radio frequency, a high-intensity focused ultrasound, etc. When a plurality of contraction areas are formed in the treatment zone, the energy-based device 200 may output location values of respective contraction centers of neighboring contraction areas such that coagulation spots are generated in an asymmetric arrangement pattern.

The energy-based device 200 may include a communication part 210, a control part 220, and an energy emission part 230.

The communication part 210 may perform communication with the image capturing device 100 which captures an image of the skin. The communication part 210 may receive the skin image obtained from the image capturing device 100. The communication part 210 may include at least one of a wired communication module and a wireless communication module.

The wired communication module may include a local area network (NAN) module, a wide area network (WAN) module, a value added network (VAN) module, etc. The wired communication module may include various cable communication modules such as a universal serial bus (USB), a high definition multimedia interface (HDMI), a digital visual interface (DVI), a recommended standard232 (RS-232), power line communication, and a plain old telephone service (POTS).

The wireless communication module may include a Wi-Fi module, a wireless broadband module, global system for mobile communication (GSM), code division multiple access (CDMA), wideband code division multiple access (WCDMA), universal mobile telecommunications system (UMTS), time division multiple access (TDMA), long term evolution (LTE), 4G, 5G, and 6G.

The control part 220 may include a memory 221 and a processor 222.

The memory 221 may store data associated with an algorithm for controlling operations of the components of the device 200 according to the present disclosure or a program for reproduction of the algorithm. The processor 222 may perform the above operation by using the data stored in the memory 221. Each of the memory 221 and the processor 222 may be implemented with an individual chip. The memory 221 and the processor 222 may be implemented with a single chip.

The memory 221 may store data supporting various functions of the device 200 according to the present disclosure and a program for the operations of the components in the device 200 according to the present disclosure, may store a plurality of input/output data, and may store various application programs (or applications) executable on the device 200 according to the present disclosure and a plurality of data and instructions for the operation of the device 200 according to the present disclosure. At least some of the application programs may be downloaded from an external server through wireless communication.

The memory 221 may include a storage medium whose type corresponds to a type of at least one of a flash memory type, a hard disk type, a solid state drive (SSD) type, a silicon disk drive (SDD) type, or a card type of memory (e.g., an SD or XD memory), a random access memory (RAM), a static random access memory (SRAM), a read-only memory (ROM), an electrically erasable programmable read-only memory (EEPROM), a programmable read-only memory (PROM), a magnetic memory, a magnetic disc, and an optical disc.

The memory 221 may store images P1, P2, P3, etc. for respective skins. The memory 221 may store data associated with asymmetrical area contraction of the skin.

The processor 222 may control the operation associated with asymmetrical area contraction of the skin. The processor 222 may include an analysis module 222a, a determination module 222b, an adjustment module 222c, and a control module 222d.

The analysis module 222a may analyze the treatment zone in the skin image received from the image capturing device 100, through the communication part 210. The analysis module 222a may further analyze an image of a grid formed in the treatment zone.

When a plurality of contraction areas are formed in the treatment zone, the determination module 222b may determine locations of contraction centers of neighboring contraction areas such that the neighboring contraction areas do not overlap each other and are formed adjacent to each other. Also, when a plurality of contraction areas are formed in the grid, the determination module 222b may further determine locations of contraction centers of neighboring contraction areas such that the neighboring contraction areas do not overlap each other and are formed adjacent to each other.

As an example, when a plurality of contraction areas are formed, the determination module 222b may determine locations of contraction centers of some contraction areas vertically neighboring to each other such that the some contraction areas vertically neighboring to each other do not overlap each other and are formed adjacent to each other.

As another example, when a plurality of contraction areas are formed, the determination module 222b may determine locations of contraction centers of some contraction areas horizontally neighboring to each other such that the some contraction areas horizontally neighboring to each other do not overlap each other and are formed adjacent to each other.

As another example, when a plurality of contraction areas are formed, the determination module 222b may determine locations of contraction centers of some contraction areas vertically and horizontally neighboring to each other such that the some contraction areas vertically and horizontally neighboring to each other do not overlap each other and are formed adjacent to each other.

The adjustment module 222c may adjust a focus location of the energy emission part 230 to face the location of each contraction center. The control module 222d may control the energy emission part 230 such that coagulation spots respectively corresponding to the locations of the contraction centers are generated in the asymmetric arrangement pattern, through the energy emission part 230.

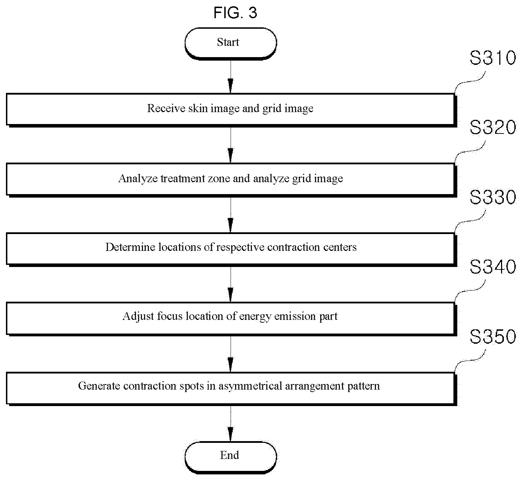

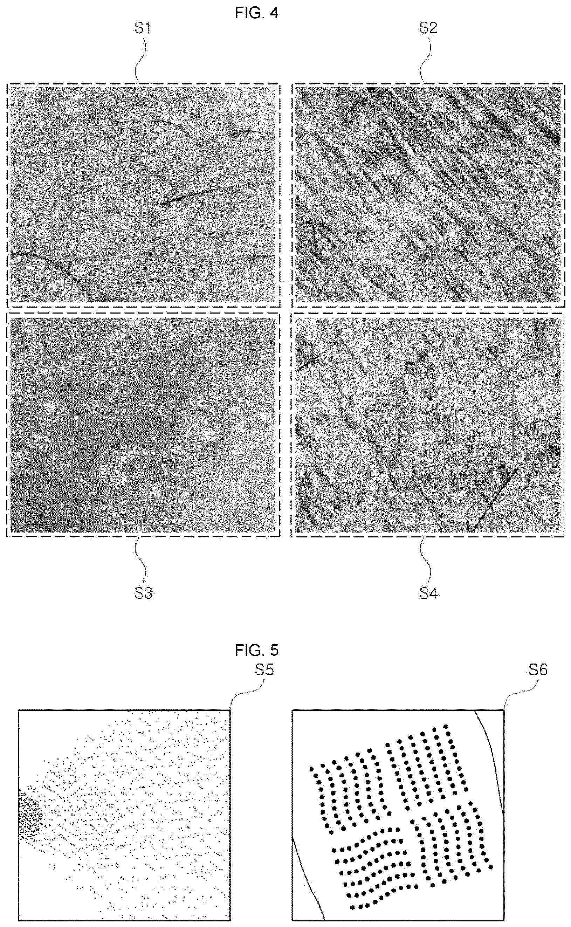

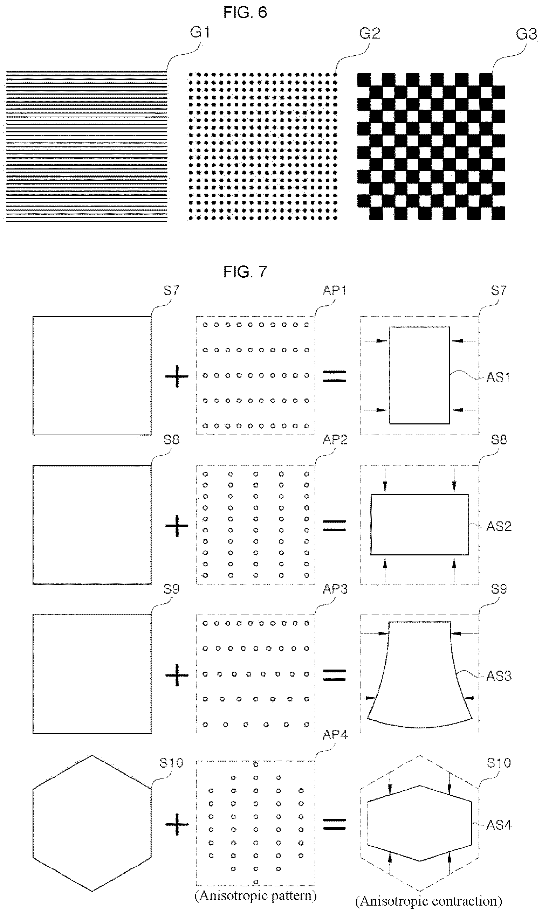

FIG. 3 is a flowchart illustrating a method of generating coagulation spots in an asymmetric arrangement pattern, according to the present disclosure. FIG. 4 is a diagram illustrating an example of a process of analyzing a treatment zone in a skin image through an analysis module of FIG. 2. FIGS. 5 and 6 are diagrams illustrating an example of a process of analyzing an image of a grid formed in a treatment zone through an analysis module.

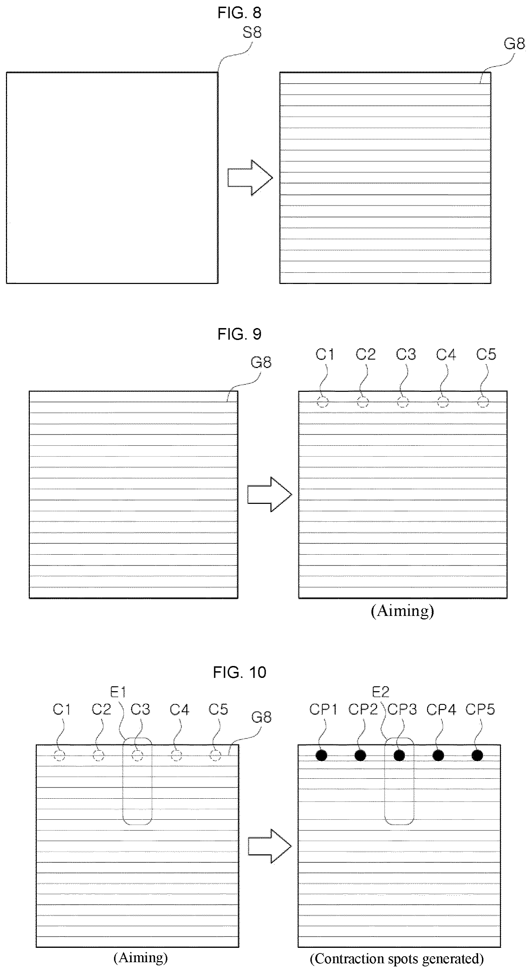

FIG. 7 is a diagram illustrating an example of a process of determining asymmetrical contraction by adjusting an asymmetrical pattern depending on anatomical location information of a treatment zone through a determination module of FIG. 2. FIGS. 8 to 17 are diagrams illustrating an example of a process of setting location values of contraction centers by using a grid in a square treatment zone from among an asymmetric pattern of FIG. 7.

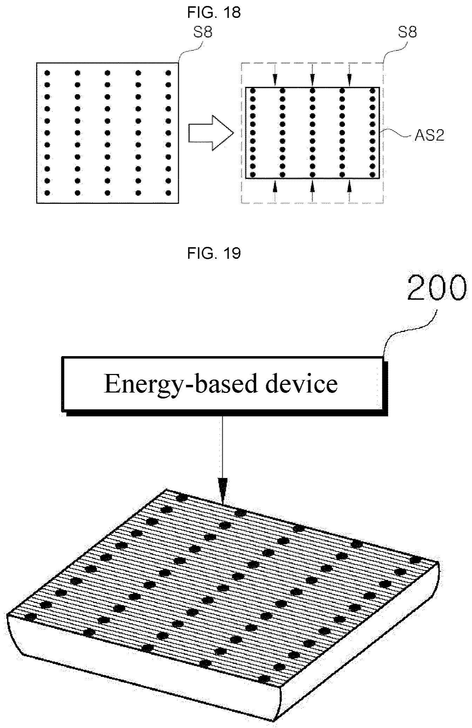

FIG. 18 is a diagram illustrating a process of setting location values of contraction centers to have a contraction area having asymmetric arrangement without a grid in a square treatment zone of an asymmetric pattern of FIG. 7. FIG. 19 is a diagram illustrating an example of a process of emitting energy such that coagulation spots are generated in an asymmetric arrangement pattern through an energy-based device of FIG. 1.

Referring to FIGS. 3 to 19, a method of generating coagulation spots in an asymmetric arrangement pattern may include a reception step S310, an analysis step S320, a determination step S330, an adjustment step S340, and a control step S350.

The reception step may receive an image of the skin from the image capturing device 100, through the communication part 210 (S310). The communication part 210 may further receive an image of the grid formed in a treatment zone from the image capturing device 100.

The analysis step may analyze a treatment zone in the skin image, through the analysis module 222a (S320). As illustrated in FIG. 4, the analysis module 222a may analyze treatment zones S1 to S4 without the grid, by using fine landmarks capable of being obtained as images of hairs, sweat glands, sebaceous glands, pigments, blood vessels, etc. As illustrated in FIG. 5, the analysis module 222a may further analyze images of grids formed in treatment zones S5 and S6. The analysis module 222a may analyze images of grids formed in the treatment zones S5 and S6 by using fine landmarks capable of being obtained as an image where the skin surface is laser-engraved, is inked, or is finely tattooed. Herein, as illustrated in FIG. 6, the shape of each of grids G1, G2, and G3 may include at least ones of a plurality of parallel lines, a plurality of points, a plurality of lines, and a plurality of surfaces.

The determination step may determine, when a plurality of contraction areas are formed in the treatment zone, locations of contraction centers of neighboring contraction areas such that the neighboring contraction areas do not overlap each other and are formed adjacent to each other, through the determination module 222b. When a plurality of contraction areas are formed in the grid, the determination module 222b may further determine locations of contraction centers of neighboring contraction areas such that the neighboring contraction areas do not overlap each other and are formed adjacent to each other.

Herein, in the case where the plurality of contraction areas are formed in the treatment zone or in the case where the plurality of contraction areas are formed in the grid, when a distance between neighboring contraction areas is long, the efficiency of area contraction decreases due to compensational dilatation in a space between contraction areas. In contrast, in the case where the plurality of contraction areas are formed in the treatment zone or in the case where the plurality of contraction areas are formed in the grid, when a distance between neighboring contraction areas is very short, neighboring contraction areas overlap each other; in this case, excessive denaturation occurs, or the efficiency of area contraction decreases.

Accordingly, to consistently contract the skin area, it is important to determine locations of contraction centers of neighboring contraction areas such that the neighboring contraction areas do not overlap each other and are formed adjacent to each other, when the plurality of contraction areas are formed in the treatment zone or when the plurality of contraction areas are formed in the grid.

As an example, when the plurality of contraction areas are formed, the determination module 222b may determine locations of contraction centers of some contraction areas vertically neighboring to each other such that the some contraction areas vertically neighboring to each other do not overlap each other and are formed adjacent to each other.

As another example, when the plurality of contraction areas are formed, the determination module 222b may determine locations of contraction centers of some contraction areas horizontally neighboring to each other such that the some contraction areas horizontally neighboring to each other do not overlap each other and are formed adjacent to each other.

As another example, when the plurality of contraction areas are formed, the determination module 222b may determine locations of contraction centers of some contraction areas vertically and horizontally adjacent to each other or locations of contraction centers of all the contraction areas such that the some contraction areas vertically and horizontally adjacent to each other do not overlap each other and are formed adjacent to each other.

That is, as illustrated in FIG. 7, the determination module 222b may determine contraction areas AS1, AS2, AS3, and AS4 for asymmetric contraction by adjusting asymmetric patterns AP1, AP2, AP3, and AP4 depending on anatomical location information of treatment zones S7, S8, S9, and S10. The anatomical location information of the treatment zones S7, S8, S9, and S10 may be at least one of contraction area information for each age group, contraction area information for each face shape, and contraction area information for each skin condition, which are recommended through the trained artificial intelligence model. The determination module 222b may determine the contraction area for optimized asymmetric contraction by using at least one of the contraction area information for each age group, the contraction area information for each face shape, and the contraction area information for each skin condition, which are based on the artificial intelligence model.

For example, the contraction area information for each age group may be contraction area information respectively corresponding to age groups such as 20s, 30s, 40s, 50s, 60s, 70s, and 80s. The contraction area information for each face shape may be contraction area information respectively corresponding to face shapes such as an egg shape, a peanut shape, a rhombus shape, a hexagon shape, a heart shape, a round shape, a long face shape, a rectangle, an inverted triangle, and an angled shape. The contraction area information for each skin condition may be contraction area information respectively corresponding to skin conditions such as a dry degree, a sensitive degree, a pigment degree, a wrinkle degree, a moisture content degree, a skin texture degree, a skin elasticity degree, a skin thickness degree, and a sebum secretion degree.

As illustrated in FIG. 8, the determination module 222b may form the grid G8 to be used as a landmark in the square treatment zone S8 of the asymmetrical pattern. As an example, the determination module 222b may form parallel lines capable of being identified as an image, by deteriorating only a partial superficial layer of the skin using the laser engraving. In this case, because only the superficial layer is deteriorated and the deep dermis layer is not affected, the skin is not contracted.

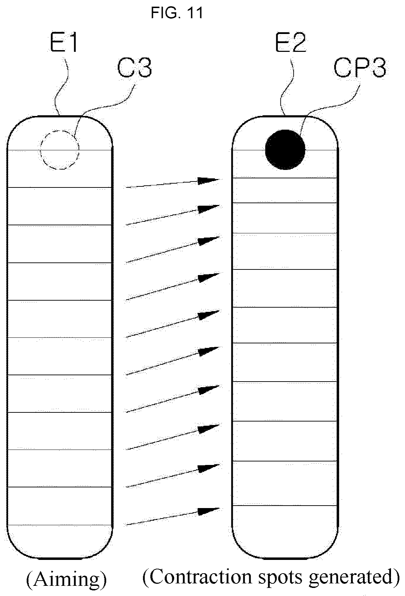

As illustrated in FIGS. 9 to 11, the determination module 222b may aim at contraction centers C1 to C5 at regular intervals on the uppermost parallel line among a plurality of parallel lines of the grid G8. As a result, as illustrated in region E1 and region E2 of FIGS. 10 and 11, parallel lines on the grid G8 are mainly drawn upward by the contraction effect of the coagulation spots CP1 to CP5 in the first line.

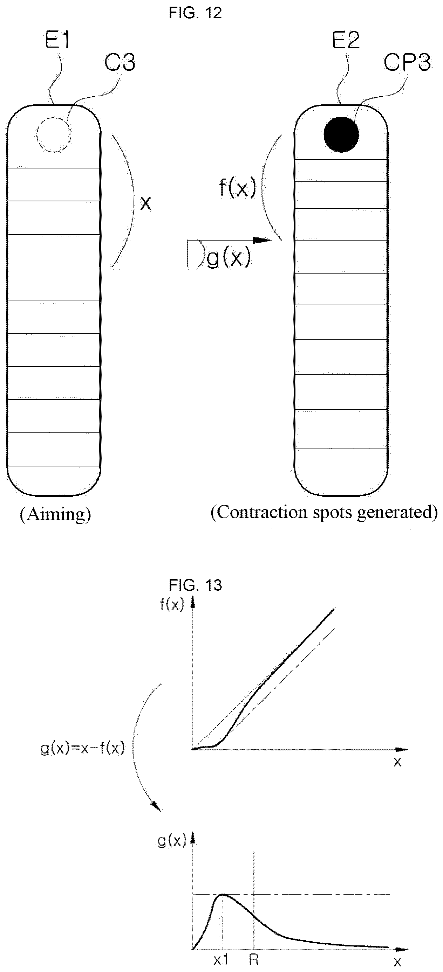

Here, as illustrated in FIGS. 12 and 13, the determination module 222b may calculate a displacement distribution g(x) in a vertically downward direction through location information of points orthogonal to the grid G8 on the virtual line passing through each of the contraction centers C1 to C5 and perpendicular to the grid G8.

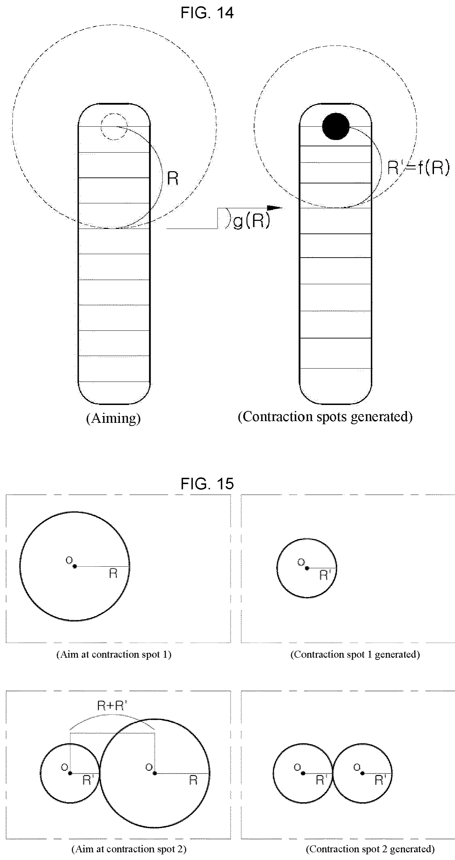

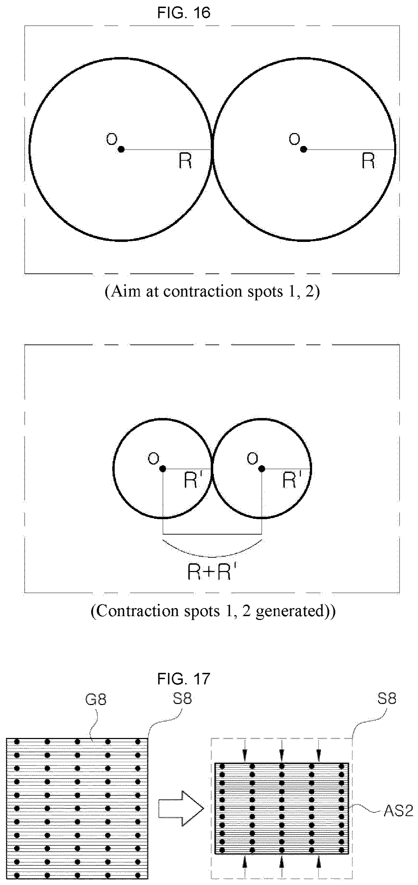

As illustrated in FIGS. 13 and 14, the determination module 222b may calculate a contraction area in the vertically downward direction based on the displacement distribution g(x) in the vertically downward direction. As illustrated in FIG. 15, the determination module 222b may sequentially form contraction centers at an interval of (R + R') in the vertically downward direction based on the contraction centers C1 to C5 of the uppermost line. That is, to generate coagulation spot 1 with the contraction center of the interval of R', the determination module 222b may aim at coagulation spot 1 with the contraction center of the R' interval; afterwards, to generate coagulation spot 2 with the contraction center of the interval of R', the determination module 222b may aim at coagulation spot 2 with the contraction center of the interval of R. As illustrated in FIG. 16, the determination module 222b may simultaneously form contraction centers of the interval of (R + R') in the vertically downward direction based on the contraction centers C1 to C5 of the uppermost line. That is, to generate coagulation spot 1 and coagulation spot 2 each having the contraction center of the interval of R', the determination module 222b may aim at coagulation spot 1 and coagulation spot 2 each having the contraction center of the interval of R.

As a result, as illustrated in FIG. 17, the square treatment zone S8 using the grid G8, which is in the shape of a smaller and flat rectangle, has the contraction area AS2 of asymmetric arrangement.

As described above, when a plurality of contraction areas are formed, the determination module 222b may determine locations of contraction centers of some contraction areas vertically neighboring to each other such that the some contraction areas do not overlap each other and are formed adjacent to each other.

The adjustment step may adjust a focus location of the energy emission part 230 through the adjustment module 222c, so as to face the location of each contraction center (S340). The energy emission part 230 may be provided as a laser, and the adjustment module 222c may adjust the focus location of the laser by using a galvanometer mirror. In addition, the adjustment module 222c may adjust the focus location of the laser by using an X-Y linear actuator.

The control step may control the energy emission part 230, through the control module 222d, such that coagulation spots respectively corresponding to the locations of the contraction centers are generated in an asymmetric arrangement pattern (S350). The control module 222d may control the energy emission part 230 such that coagulation spots respectively corresponding to the locations of the contraction centers are generated in the asymmetric arrangement pattern, through the energy emission part 230. As illustrated in FIG. 18, the energy-based device 200 may emit energy such that the coagulation spots respectively corresponding to the locations of the contraction centers are generated in the asymmetric arrangement pattern. As illustrated in FIG. 19, the energy-based device 200 may set location values of the contraction centers to the treatment zone S8, so as to have the contraction area AS2 whose asymmetric arrangement is previously set without the grid.

As described above, according to the present disclosure, it is possible to consistently contract the skin area as expected regardless of the operator's experience and intuition and to contract the treatment zone to the skin area and shape optimized depending on the anatomical location information of the treatment zone.

In the specification, an energy-based device according to the present disclosure includes all of various devices capable of providing a result to the user by performing computational processing. For example, the energy-based device according to the present disclosure may include all of a computer, a server device, and a portable terminal or may be implemented in the form of any one thereof.

Herein, the computer may include, for example, a notebook, a desktop, a laptop, a tablet PC, a slate PC, etc., which is equipped with a web browser.

The server device which is a server processing information through the communication with an external device may include an application server, a computing server, a database server, a file server, a mail server, a proxy server, a web server, etc.

For example, the portable terminal which is a wireless communication device ensuring portability and mobility may include all types of handheld-based wireless communication devices such as a personal communication system (PCS), global system for mobile communications (GSM), a personal digital cellular (PDC), a personal handyphone system (PHS), personal digital assistant (PDA), international mobile telecommunication (IMT)-2000, code division multiple access (CDMA)-2000, wideband code division multiple access (WCDMA), a wireless broadband Internet (WiBro) terminal, and a smartphone, and a wearable device such as a watch, a ring, a bracelet, an anklet, a necklace, glasses, a contact lens, or a head-mounted device (HMD).

At least one component may be added or deleted to correspond to performance of the components illustrated in FIGS. 1 and 2. Also, it will be easily understood by one skilled in the art that mutual locations of the components are capable of being changed to correspond to system performance or structure.

FIG. 3 describes a plurality of steps as being executed sequentially. However, this is provided only as an example to describe the technical idea of the present embodiment. One skilled in the art to which the present embodiment pertains may apply various revision and modification such that the order described in FIG. 3 is changed and executed or one or more of the plurality of steps are executed in parallel, without departing from essential characteristics of embodiments of the present disclosure. Accordingly, FIG. 3 is not limited to a time-series order.

Meanwhile, the disclosed embodiments may be implemented in the form of a recording medium storing instructions executable by a computer. The instructions may be stored in the form of a program code, and the instructions, when executed by a processor, may generate a program module to perform the operations of the disclosed embodiments. The recording medium may be implemented with a computer-readable recording medium.

The computer-readable recording medium includes all types of recording media storing instructions capable of being interpreted by the computer. For example, there may be a read only memory (ROM), a random access memory (RAM), a magnetic tape, a magnetic disk, a flash memory, an optical data storage device, etc.

The embodiments are described above with reference to the accompanying drawings. One skilled in the art to which the present disclosure pertains will understand that the present disclosure may be implemented in forms different from those of the disclosed embodiments without changing the technical idea or essential features of the present disclosure. The disclosed embodiments are provided as an example and should not be construed as limiting.

## Claims

1. An energy-based device which generate coagulation spots in an asymmetric arrangement pattern, comprising:
a communication part configured to perform communication with an image capturing device capturing an image of a skin; and
a processor configured to control an operation associated with asymmetrical area contraction of the skin,
wherein the processor includes:
an analysis module configured to analyze a treatment zone in the skin image received from the image capturing device, through the communication part;
a determination module, wherein, when a plurality of contraction areas are formed in the treatment zone, the determination module determines locations of contraction centers of contraction areas neighboring to each other such that the neighboring contraction areas do not overlap each other and are formed adjacent to each other;
an adjustment module configured to adjust a focus location of an energy emission part, so as to face the location of each of the contraction centers; and
a control module configured to control the energy emission part such that coagulation spots respectively corresponding to the locations of the contraction centers are generated in the asymmetric arrangement pattern through the energy emission part.

2. The energy-based device of claim 1, wherein the analysis module further analyzes an image of a grid formed in the treatment zone.

3. The energy-based device of claim 2, wherein, when the plurality of contraction areas are formed in the grid, the determination module further determines locations of contraction centers of the neighboring contraction areas, such that the neighboring contraction areas do not overlap each other and are formed adjacent to each other.

4. The energy-based device of claim 1 or 3, wherein, when the plurality of contraction areas are formed, the determination module determines locations of contraction centers of the some contraction areas vertically neighboring to each other, such that some contraction areas vertically neighboring to each other do not overlap and are formed adjacent to each other.

5. The energy-based device of 1 or claim 3, wherein, when the plurality of contraction areas are formed, the determination module determines locations of contraction centers of the some contraction areas horizontally neighboring to each other, such that some contraction areas horizontally neighboring to each other do not overlap and are formed adjacent to each other.

6. The energy-based device of claim 1 or 3, wherein, when the plurality of contraction areas are formed, the determination module determines locations of contraction centers of the some contraction areas vertically and horizontally neighboring to each other such that some contraction areas vertically and horizontally neighboring to each other do not overlap and are formed adjacent to each other.

7. The energy-based device of claim 2, wherein a shape of the grid is at least ones of a plurality of points, a plurality of lines, and a plurality of surfaces.

8. A method of generating coagulation spots in an asymmetric arrangement pattern, which is performed by an energy-based device, the method comprising:
receiving an image of a skin from an image capturing device, through a communication part of the energy-based device;
analyzing a treatment zone in the skin image, through an analysis module of the energy-based device;
determining, when a plurality of contraction areas are formed in the treatment zone, locations of contraction centers of contraction areas neighboring to each other such that the neighboring contraction areas do not overlap each other and are formed adjacent to each other, through a determination module of the energy-based device;
adjusting a focus location of an energy emission part of the energy-based device to face the location of each of the contraction centers, through an adjustment module of the energy-based device; and
controlling the energy emission part such that coagulation spots respectively corresponding to the locations of the contraction centers are generated in the asymmetric arrangement pattern, through a control module of the energy-based device.

9. The method of claim 8, wherein the analyzing further analyzes an image of a grid formed in the treatment zone, through the analysis module.

10. The method of claim 9, wherein, when the plurality of contraction areas are formed in the grid, the determining further determines locations of contraction centers of the neighboring contraction areas, such that the neighboring contraction areas do not overlap each other and are formed adjacent to each other, through the determination module.

11. The method of any one of claim 8 or 10, wherein, when the plurality of contraction areas are formed, the determining determines locations of contraction centers of the some contraction areas vertically neighboring to each other, such that some contraction areas vertically neighboring to each other do not overlap and are formed adjacent to each other, through the determination module.

12. The method of any one of claim 8 or 10, wherein, when the plurality of contraction areas are formed, the determining determines locations of contraction centers of the some contraction areas horizontally neighboring to each other, such that some contraction areas horizontally neighboring to each other do not overlap and are formed adjacent to each other, through the determination module.

13. The method of any one of claim 8 or 10, wherein, when the plurality of contraction areas are formed, the determining determines locations of contraction centers of the some contraction areas vertically and horizontally neighboring to each other, such that some contraction areas vertically and horizontally neighboring to each other do not overlap and are formed adjacent to each other, through the determination module.

14. A computer-readable recording medium coupled to a computer, which is hardware, to store a program for executing the method of generating the coagulation spots in the asymmetric arrangement pattern of any one of claims 8-13.

15. A system which generates coagulation spots in an asymmetric arrangement pattern, comprising:
an image capturing device configured to capture an image of a skin; and
an energy-based device configured to perform communication with the image capturing device and to control an operation associated with asymmetrical area contraction of the skin,
wherein the energy-based device includes:
an analysis module configured to analyze a treatment zone in the skin image received from the image capturing device;
a determination module, wherein, when a plurality of contraction areas are formed in the treatment zone, the determination module determines locations of contraction centers of contraction areas neighboring to each other such that the neighboring contraction areas do not overlap each other and are formed adjacent to each other;
an adjustment module configured to adjust a focus location of an energy emission part, so as to face the location of each of the contraction centers; and
a control module configured to control the energy emission part such that coagulation spots respectively corresponding to the locations of the contraction centers are generated in the asymmetric arrangement pattern, through the energy emission part.

16. The system of claim 15, wherein the determination module further determines a contraction area for optimized asymmetric contraction by using at least one of contraction area information for each age group, contraction area information for each face shape, and contraction area information for each skin condition, which are based on an artificial intelligence model.
